Europäisches Patentamt
European Patent Office
Office européen des brevets

(11) Numéro de publication: **0 122 353**
**A1**

# (12) DEMANDE DE BREVET EUROPEEN

(21) Numéro de dépôt: 83400730.4

(22) Date de dépôt: 12.04.83

(51) Int. Cl.³: **A 61 B 17/12**

(43) Date de publication de la demande:
24.10.84 Bulletin 84/43

(84) Etats contractants désignés:
DE GB IT SE

(71) Demandeur: NOMEL
Tour Franklin Quartier Boieldieu
F-92081 Puteaux-La Défense(FR)

(72) Inventeur: Hrouda, Georges
42, rue du Cherche-Midi
F-75006 Paris(FR)

(72) Inventeur: Dufour, Serge
12, rue Kruger
F-91100 Corbeil Essonnes(FR)

(74) Mandataire: Beauchamps, Georges et al,
Cabinet Z.Weinstein 20, avenue de Friedland
F-75008 Paris(FR)

(54) Dispositif de distribution de pinces hémostatiques, par exemple dans un dispositif d'application de pinces.

(57) Le dispositif distributeur (1) de pinces hémostatiques (2) dans un dispositif d'application (20) desdites pinces comprend deux parties (3, 4) montées pivotantes autour d'un axe de rotation (5), les pinces hémostatiques (2) étant disposées dans un réservoir (11) de la première partie (4) et amenées sélectivement dans une ouverture de distribution (13) débouchant dans un logement (12) de réception du dispositif d'application (20). La pince hémostatique (2) est positionnée entre les mâchoires du dispositif d'application (20) par un élément d'extraction (17) solidaire de la seconde partie (3).

L'invention s'applique notamment dans le domaine médical.

Fig. 1

EP 0 122 353 A1

1

<u>Dispositif de distribution de pinces hémostatiques,</u>
<u>par exemple dans un dispositif d'application de</u>
<u>pinces</u>

La présente invention concerne un dispositif de
distribution de pinces hémostatiques par exemple dans
un dispositif d'application de pinces.

Elle vise plus particulièrement un dispositif
distributeur de pinces hémostatiques pour la ligature
rapide de vaisseaux sanguins nécessitée en chirurgie,
lors de l'ablation d'un organe par exemple, ledit
distributeur étant d'une utilisation aisée et sûre
pour le chirurgien qui prélève lesdites pinces au
moyen d'un dispositif d'application de pinces.

L'utilisation de pinces hémostatiques pour serrer et
obturer des vaisseaux sanguins pendant une opération
chirurgicale est bien connue dans la pratique, et de
nombreux supports de pinces, dispositifs d'application
de pinces et de pinces hémostatiques ont fait l'objet
de nombreux brevets antérieurs.

Par exemple, le brevet Français N° 71 18 034 déposé
le 18 Mai 1971, décrit un support de pinces hémostatiques, un procédé de positionnement de pinces dans
ledit support et un procédé de retrait de ces pinces
par un dispositif d'application en forme de forceps.

0122353

2

Toutefois, pour prélever une pince hémostatique sur ce support connu, le chirurgien doit prendre dans une main ce support et dans l'autre main le dispositif d'application pour introduire les extrémités des mâchoires de ce dispositif d'application dans ledit support pour positionner la pince entre ces mâchoires. De plus, plusieurs pinces hémostatiques sont disposées sur le support, et dans le cas très fréquent où le chirurgien n'utilise pas la totalité des pinces hémostatiques disposées sur ce support, les pinces non utilisées sont jetées car il est difficile avec un tel support de re-stériliser ces pinces pour une nouvelle utilisation. Cette non utilisation de pinces hémostatiques est un important inconvénient compte tenu du prix élevé de ces dispositifs.

Par ailleurs, il est nécessaire de prévoir un support de forme particulière pour chaque profil de pince hémostatique, et pour chaque dispositif d'application c'est-à-dire le profil des extrémités des mâchoires de ce dispositif.

La présente invention a pour but de remédier à tous ces inconvénients en proposant un dispositif de distribution de pinces hémostatiques par exemple dans un dispositif d'application de pinces permettant au chirurgien de prélever dans le dispositif distributeur les pinces une par une par simple positionnement du dispositif d'application dans ledit distributeur monté de manière fixe sur un support.

A cet effet, l'invention a pour objet un dispositif destiné à distribuer sélectivement des pinces hémostatiques, par exemple dans un dispositif d'application de pinces caractérisé en ce qu'il comprend deux parties montées mobiles l'une par rapport à l'autre par action contre une force de rappel, ladite première partie comprenant un support-guide de pinces hémostatiques et un logement de forme adaptée pour recevoir, guider et centrer au moins les extrémités des mâchoires dudit dispositif d'application de pinces, ledit logement présentant dans sa paroi de fond une ouverture de distribution, et des moyens pour amener sélectivement les pinces en regard de ladite seconde partie comprenant un élément d'extraction en forme de lame agencé pour pénétrer dans ladite ouverture de distribution, pour extraire la pince hémostatique sélectivement amenée, par mouvement de l'une des première ou seconde partie vers l'autre.

Ainsi, le chirurgien peut prélever une pince hémostatique simplement par disposition du dispositif d'application de pinces et action sur l'une des parties du support-guide par l'intermédiaire du dispositif d'application de pinces, c'est-à-dire ce dispositif permet au chirurgien de prélever des pinces hémostatiques avec une seule main.

Selon un premier mode de réalisation lesdites première partie et deuxième partie sont montées pivotantes l'une par rapport à l'autre.

Selon un deuxième mode de réalisation, lesdites première partie et deuxième partie sont montées déplaçables parallèlement l'une par rapport à l'autre.

Selon une autre caractéristique de l'invention, la forme de l'extrémité de l'élément d'extraction entrant en contact avec la pince hémostatique est sensiblement conjuguée au profil désiré de la pince hémostatique avant son application, ledit élément d'extraction formant la pince hémostatique selon ce profil désiré pendant son extraction et sa distribution dans le dispositif d'application précité.

Ainsi, il est possible d'alimenter le dispositif de distribution de l'invention avec des pinces hémostatiques constituées simplement par un segment droit de matériau adapté pour la formation de pinces hémostatiques, c'est-à-dire présentant des propriétés élastiques. Avantageusement, ce segment droit sera préformé, par exemple par un point d'amorce de pliage.

Selon encore une autre caractéristique de l'invention, la première partie précitée formant support-guide du dispositif de distribution de pinces hémostatiques comprend un premier élément formant support pour les pinces hémostatiques, ayant une section transversale sensiblement conjuguée au profil des pinces chargées dans ledit dispositif, et un second élément formant enveloppe partielle dudit premier élément et définissant avec ce dernier un espace formant réservoir pour les pinces, le logement précité de centrage, et réception du dispositif d'application de pinces étant formé dans ledit second élément formant enveloppe.

Avantageusement, les premier et second éléments précités sont montés pivotants l'un par rapport à l'autre pour permettre le positionnement et le chargement des pinces hémostatiques dans le réservoir défini entre ces deux éléments. Les pinces hémostatiques chargées dans le dispositif se présentent avantageusement sous forme de bandes de pinces ou cartouches constituées par une pluralité de pinces assemblées, par exemple, par collage les unes aux autres.

Par ailleurs, le logement précité de guidage et réception du dispositif d'application de pinces comprend deux gorges de profils conjugués respectivement aux extrémités des mâchoires du dispositif d'application de pinces pour ainsi permettre une distribution et un positionnement corrects de la pince hémostatique dans ledit dispositif d'application de pinces. Ainsi, dans le cas d'une fausse manipulation, c'est-à-dire d'un positionnement incorrect du dispositif d'application de pinces dans ledit logement, il sera impossible au chirurgien de prélever une pince hémostatique.

D'autres caractéristiques, détails et avantages de l'invention apparaîtront plus clairement à la lumière de la description explicative qui va suivre faite en référence aux dessins schématiques annexés, donnés uniquement à titre d'exemple, et dans lesquels :

La figure 1 est une vue schématique en coupe d'un premier mode de réalisation préféré d'un dispositif de distribution de pinces hémostatiques conforme à la présente invention, un dispositif d'application de pinces étant positionné dans son logement de réception.

La figure 2 est une coupe selon la ligne II-II de la figure 1.

La figure 3 est une vue de dessus avec arrachement partiel selon la flèche III de la figure 1, un dispositif d'application de pinces n'étant pas positionné dans son logement de réception.

La figure 4 est une vue en coupe identique à la figure 1, illustrant la position du dispositif de distribution de pinces hémostatiques lors de la distribution d'une pince dans le dispositif d'application de pinces, et

La figure 5 est une vue en coupe analogue à celle de la figure 2 d'un deuxième mode de réalisation préféré d'un dispositif de distribution de pinces hémostatiques conforme à la présente invention dans lequel
les parties du dispositif sont déplaçables l'une par
rapport à l'autre.

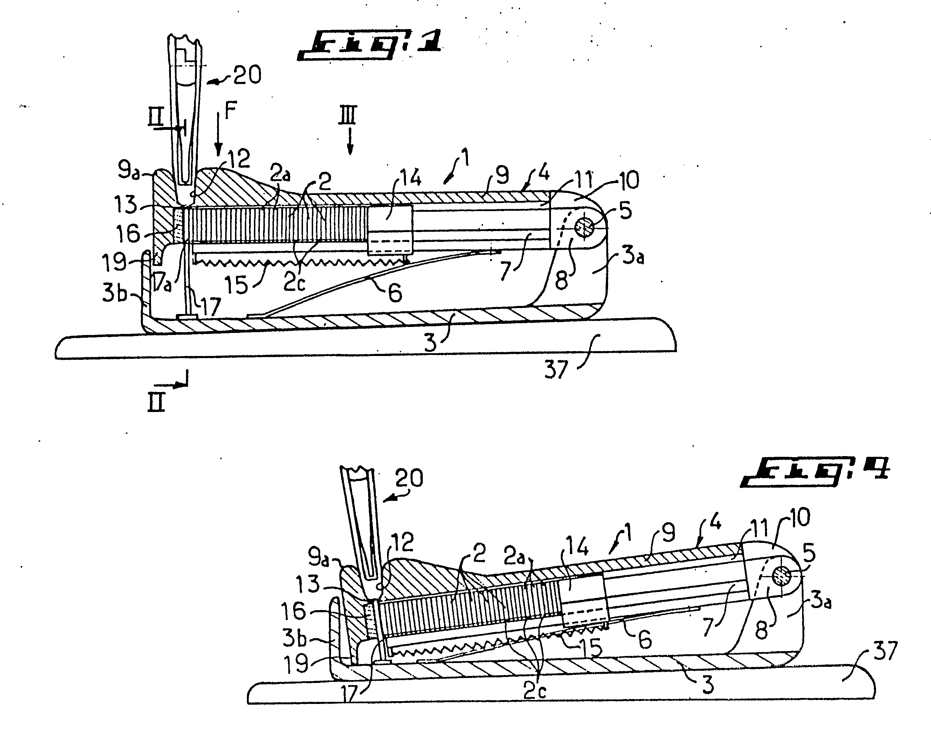

En se référant aux figures 1 à 4, le dispositif 1 de
distribution sélective de pinces hémostatiques 2
comprend deux parties 3, 4 montées mobiles l'une par
rapport à l'autre. De préférence, et selon le mode
de réalisation représenté, les deux parties 3, 4 sont
montées pivotantes l'une par rapport à l'autre autour
d'un axe de pivotement 5 par action contre une force
de rappel telle que des éléments de ressort 6 fixés
par l'une de leurs extrémités sur la première partie 4
et prenant appui par leur autre extrémité sur la
seconde partie 3.

La seconde partie 3 formant support pour le dispositif
de distribution 1 comprend à une de ses extrémités
des parois 3a sensiblement perpendiculaires audit
élément 3 et supportant l'axe de rotation 5.

Avantageusement, elle comprend des parois latérales
pour former une paroi de protection 3b.

La première partie 4 du dispositif de distribution 1
de l'invention comprend un support-guide 7 de forme
allongée et monté pivotant autour de l'axe 5 de
pivotement par deux pattes de support 8. Selon l'invention, le support-guide 7 a une surface supérieure
de forme sensiblement conjuguée au profil des pinces
hémostatiques 2. Ce support-guide peut être soit une
barre profilée métallique ou non ou un élément obtenu
par un pliage d'une bande de tôle ou par usinage d'un
barreau.

En outre, cette première partie 4 du dispositif de distribution 1 de l'invention comprend un second élément 9 monté articulé par rotation autour de l'axe de pivotement 5 par des pattes de support 10. Cet élément 9 a un profil interne sensiblement conjugué à la surface supérieure de l'élément 7 de manière à venir s'emboîter sur ledit élément 7 pour ainsi former une enveloppe de cet élément. L'espace défini 11 entre les éléments 9 et 7 constitue un espace réservoir 1 ou un magasin pour les pinces hémostatiques 2.

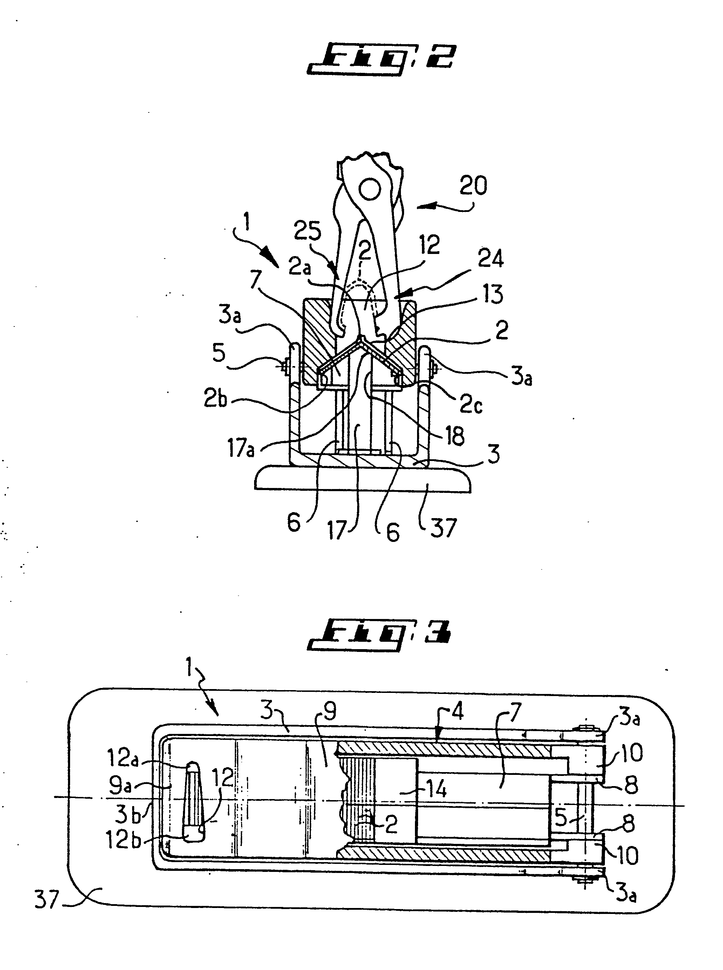

Avantageusement, chaque pince hémostatique 2 se compose d'une lame flexible présentant sensiblement en sa partie centrale une amorce de pliage 2a de façon à définir deux branches formant entre elles un angle relativement ouvert avec deux parties extrêmes 2c, 2b libres. Une de ces parties extrêmes, par exemple 2b étant recourbée soit en forme de boucle, soit pour former un crochet, comme illustré à la figure 2.

Avantageusement, ces pinces hémostatiques 2 sont disposées alignées dans l'espace 11, sur le support-guide 7 et sont alimentées sous forme de cartouches obtenues par, par exemple, collage des pinces hémostatiques l'une contre l'autre, d'une manière analogue aux cartouches d'agrafes pour agrafeuses.

Selon l'invention, et avantageusement, l'élément formant enveloppe 9 présente au voisinage de son extrémité libre 9a un logement 12, présentant un profil adapté et notamment des gorges latérales longitudinales 12a, 12b de dimensions déterminées pour recevoir, centrer et guider de manière très précise les extrémités des mâchoires d'un dispositif d'application 20 de pinces hémostatiques.

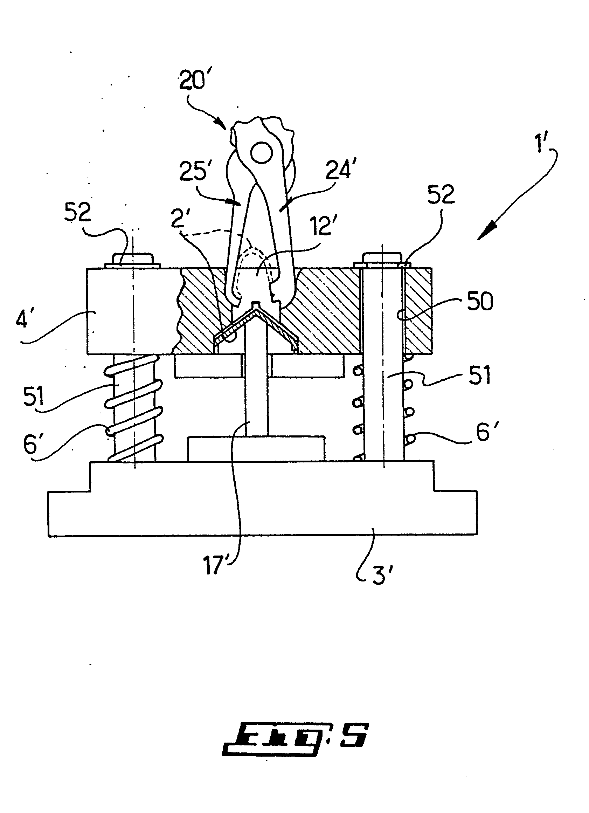

Comme illustré à la figure 3, avantageusement, les gorges latérales 12a, 12b ont des dimensions différentes, et dans le mode de réalisation préféré de l'invention, la gorge 12b a une section plus grande que la gorge 12a pour recevoir un dispositif d'application 20. Ainsi, le dispositif d'application 20 de pinces hémostatiques ne peut être introduit que selon une position bien déterminée dans le logement 12 de réception. En outre, ce logement de réception 12 comprend dans sa paroi de fond une fente 13 pour permettre le passage d'une pince hémostatique 2 et ainsi son positionnement entre les mâchoires du dispositif d'application de pinces 20, comme illustré à la figure 2.

Dans le mode de réalisation préféré de l'invention, le dispositif de distribution 1 comprend des moyens pour amener sélectivement les pinces hémostatiques 2 en regard de l'ouverture de distribution 13. Ces moyens sont constitués par une butée 14 montée coulissante sur le support-guide 7 et actionnée vers l'extrémité du support-guide 7 par un ressort 15. Par ailleurs, pour éviter que sous l'action de ce ressort les pinces hémostatiques soient éjectées hors du support-guide au niveau de l'extrémité 7a, cette extrémité présente une butée 16 qui peut être, dans un mode de réalisation préféré de l'invention, montée fixement ou venant de matière avec l'élément support-guide 7.

Il est bien entendu que ce mode de réalisation des moyens d'amenée sélective des pinces en regard de l'ouverture 13 est donné simplement à titre d'exemple et que tous moyens techniques équivalents peuvent être utilisés sans pour cela sortir du cadre de l'invention.

Conformément à l'invention et pour permettre d'extraire et de distribuer sélectivement une pince hémostatique dans un dispositif d'application 20, un élément d'extraction 17, en forme de lame est fixé sur la partie support 3, sensiblement en regard de l'ouverture de distribution 13, pour ainsi lors du pivotement de la partie 4 vers la partie 3 sous l'action d'une force de poussée représentée par la flèche F, et exercée avantageusement par l'intermédiaire du dispositif d'application 20, entraîner une pince hémostatique 2 entre les mâchoires du dispositif d'application 20, comme illustré en traits pointillés à la figure 2, en traversant d'une part une ouverture 18 pratiquée sur le support-guide 7 immédiatement en amont de l'élément de butée de l'extrémité 16, et à travers l'ouverture de distribution 13 du logement 12, comme illustré à la figure 4.

Avantageusement, la lame 17 est relativement flexible pour ainsi être légèrement incurvée lors du déplacement de la première partie 4, du dispositif distributeur 1 de l'invention vers la partie support 3.

Par ailleurs, l'extrémité 17a de la lame 17 a une forme sensiblement conjuguée au profil, illustré en traits pointillés à la figure 2, que l'on désire donner à la pince hémostatique avant son application sur un conduit souple tel qu'un vaisseau sanguin par exemple. Ainsi, lors de l'application de la lame 17 pour déplacer la pince hémostatique 2 depuis le logement 11, entre les mâchoires du dispositif d'application de pinces 20, cette lame déforme la pince pour la conformer au profil désiré. Ainsi, il est possible de disposer dans ledit dispositif de distribution 1 des pinces hémostatiques 2 comprenant simplement une amorce de pliage 2a. Ceci permet de faciliter la fabrication de ces pinces hémostatiques 2.

Par ailleurs, dans le cas où la pince hémostatique n'est pas symétrique par rapport à son amorce de pliage 2a, le profil du logement 11 défini par le support-guide 7 et l'élément formant enveloppe 9 est conjugué au profil de la pince hémostatique 2. Ainsi, il n'existe qu'une seule position pour mettre en place les cartouches de pinces hémostatiques 2 dans le logement 11.

De ce fait, par la forme particulière du logement 12 de réception du dispositif d'application 20, et du logement 11 des cartouches de pinces hémostatiques, il est impossible de positionner une pince hémostatique 2 dans le dispositif d'application 20 d'une manière incorrecte, par exemple par inversion des branches ou du dispositif d'application 20.

Bien entendu, dans le cas d'utilisation de pinces hémostatiques symétriques et d'un dispositif d'application 20 comportant des extrémités de mâchoires identiques, ce problème n'existe pas.

Par ailleurs, il est avantageux que l'élément formant enveloppe 9 présente à son extrémité 9a un élément formant saillie 19 en direction de l'élément formant support 3 pour limiter le mouvement de pivotement de la partie 4, et ainsi déterminer la profondeur d'insertion de la pince hémostatique 2 dans les mâchoires du dispositif d'application 20.

De plus, un des avantages du dispositif de distribution de la présente invention réside dans le fait qu'il peut être fixé sur une table ou un socle 37 amovible ou non, par fixation de la partie 3 formant support. Ainsi, le chirurgien tenant le dispositif d'application de pinces 20 d'une main peut par simple insertion des extrémités des mâchoires de ce dispositif 20 dans le logement de réception 12, cette insertion étant obligatoirement correcte, et par application d'une poussée F
sur ledit dispositif d'application 20, provoquant le
pivotement de la partie 4 du dispositif de distribution 1 de l'invention comme illustré à la figure 4,
vers l'élément de support 3, introduire une pince
hémostatique 2 entre les mâchoires du dispositif
d'application 20. Simultanément à cette opération, la
pince hémostatique 2 sera formée au profil désiré par
la lame d'extraction 17.

En référence à la figure 5, on a représenté un deuxième
mode de réalisation d'un dispositif de distribution de
l'invention et pour lequel on a utilisé les mêmes
numéros de référence accompagnés du signe prime pour
les pièces essentiellement identiques à celles du
premier mode de réalisation des figures 1 à 4. Ainsi,
selon ce deuxième mode de réalisation de la figure 5,
on peut constater que la première partie 4' est montée
déplaçable par rapport à la seconde partie 3' formant
support pour le dispositif de distribution 1' grâce
à la présence dans la masse de la première partie 4'
de préférence d'au moins deux ouvertures 50 destinées
à être insérées autour de montants ou colonnes de
guidage 51 de manière à servir au guidage du déplacement de la première partie 4' relativement à la
deuxième partie 3'. Naturellement la forme des montants 51 est indifférente et est par exemple cylindrique comme représenté. En outre, comme dans le cas du
premier mode de réalisation, la première partie 4'
est normalement repoussée dans sa position la plus
éloignée de la seconde partie 3' à l'aide de moyens
de rappel tels que des éléments de ressort 6' agencés
autour des montants 51 entre lesdites première partie
4' et seconde partie 3'. Ces montants 51 sont avantageusement fixés sur la seconde partie 3' ou peuvent
faire corps avec celle-ci. Naturellement également, on peut prévoir des butées d'arrêt 52, pour limiter le déplacement de la première partie 4' relativement à la seconde partie 3', aux extrémités libres des montants 51. Ces butées d'arrêt 52 peuvent être amovibles et être constituées par exemple par tout moyen approprié tel qu'un écrou vissé sur une partie filetée correspondante de l'extrémité libre du montant 51. Naturellement, on peut prévoir autant de montants 51 que désiré pour un fonctionnement correct du dispositif.

Ainsi, la présente invention propose un ensemble d'appareils pouvant être utilisé par exemple lors d'une intervention chirurgicale, pour l'application de pinces hémostatiques notamment pour la ligature de vaisseaux, permettant une distribution sûre et très simple de pinces hémostatiques dans un dispositif d'application de pinces.

Le dispositif de distribution de l'invention permet, en fin d'opération chirurgicale, une re-stérilisation efficace et sûre de l'ensemble du dispositif qui est essentiellement constitué d'éléments métalliques et des pinces hémostatiques restantes dans le magasin 11. En effet, il est possible d'enlever les pinces hémostatiques du logement 11, sans les déformer, et les re-stériliser de manière sûre.

Bien entendu, l'invention n'est nullement limitée au mode de réalisation du dispositif de distribution de pinces décrit et représenté qui n'a été donné qu'à titre d'exemple. C'est ainsi que la forme du support-guide 7, de l'élément formant enveloppe 9 du dispositif de distribution 1 est adaptée au profil de la pince hémostatique utilisée. C'est dire que l'invention comprend tous les moyens constituant des équivalents techniques des moyens décrits ainsi que leurs combinaisons.

Revendications

1. Dispositif destiné à distribuer sélectivement des pinces hémostatiques, par exemple, dans un dispositif d'application de pinces, comprenant deux parties montées mobiles l'une par rapport à l'autre par action contre une force de rappel, une première partie comportant un logement pourvu dans sa paroi de fond d'une ouverture de distribution, pour guider et recevoir au moins les extrémités des mâchoires dudit dispositif à l'application de pinces et un support-guide pour supporter et amener une à une les pinces hémostatiques en regard de ladite ouverture de distribution, une seconde partie comportant un élément d'extraction pour amener ladite pince en regard de ladite ouverture de distribution entre les mâchoires du dispositif d'application de pinces, caractérisé en ce que ledit élément d'extraction (17) est en forme de lame agencée pour pénétrer dans ladite ouverture (13) de distribution par mouvement de l'une des première ou seconde parties (3, 4) vers l'autre, la forme de l'extrémité (18) dudit élément d'extraction (17) est sensiblement conjuguée au profil désiré de la pince hémostatique (2) pour son application au moyen du dispositif applicateur précité, ladite pince (2) étant conformée selon ledit profil pendant son extraction et sa distribution dans ledit dispositif applicateur (20) de pinces.

2. Dispositif selon la revendication 1, caractérisé en ce que la première partie (4) précitée comportant le support-guide (7, 9) comprend un premier élément (7) formant support pour les pinces hémostatiques (2), et ayant une section transversale sensiblement conjuguée au profil des pinces chargées dans ledit dispositif (1), et un second élément (9) formant enveloppe partielle dudit premier élément (7) et définissant avec ce dernier un espace (11) formant magasin pour les pinces (2), le logement (12) précité de centrage et de réception du dispositif applicateur (20) précité étant formé dans ledit second élément (9).

3. Dispositif selon la revendication 1 ou 2, caractérisé en ce que la première partie (4) et la seconde partie (3) précitées sont montées pivotantes l'une par rapport à l'autre.

4. Dispositif selon la revendication 2 ou 3, caractérisé en ce que le premier élément (7) précité et le second élément (9) précité sont montés pivotants l'un par rapport à l'autre, pour permettre le positionnement des pinces hémostatiques (2) dans le logement (11) précité.

5. Dispositif selon la revendication 1 ou 2, caractérisé en ce que la première partie (4) et la seconde partie (3) sont montées déplaçables parallèlement l'une relativement à l'autre.

6. Dispositif selon l'une des revendications précédentes, caractérisé en ce que le second élément (9) précité formant enveloppe comprend une paroi recouvrant l'extrémité libre du premier élément (7) précité, ladite paroi comportant un prolongement formant saillie en direction de la seconde partie (3) du dispositif pour former une butée de fin de course desdites première et seconde parties (3; 4) l'une vers l'autre.

7. Dispositif selon l'une des revendications précédentes, caractérisé en ce que la seconde partie (3) précitée est montée fixement sur un socle formant support (37).

8. Dispositif selon l'une des revendications précédentes, caractérisé en ce que les pinces hémostatiques précitées sont chargées dans ledit logement (11) sous forme de bandes de pinces ou cartouches constituées par une pluralité de pinces (2) assemblées notamment par collage les unes aux autres.

Fig. 1

Fig. 4

## Fig. 2

## Fig. 3

0122353

Fig. 5

Office européen
des brevets

**RAPPORT DE RECHERCHE EUROPEENNE**

**0122353**
Numéro de la demande

EP 83 40 0730

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | US-A-3 598 281 (M. WATERMEIER) <br> * En entier * | 1-3,8 | A 61 B 17/12 |
| A | US-A-3 170 596 (C. NYBERG) <br> * Figures; colonne 1, ligne 54 - colonne 2, ligne 56 * | 1-4,7 | |
| A | EP-A-0 072 171 (ETHICON INC.) <br> * Figures * | 1 | |
| D,A | FR-A-2 091 762 (CODMAN & SHURTLEFF) <br> * En entier * | 1 | |

DOMAINES TECHNIQUES RECHERCHES (Int. Cl. ³)

A 61 B

Le présent rapport de recherche a été établi pour toutes les revendications

| Lieu de la recherche | Date d'achèvement de la recherche | Examinateur |
|---|---|---|
| LA HAYE | 23-12-1983 | LEVENBACH J.H. |